# EUROPEAN PATENT APPLICATION

(11) **EP 4 467 158 A2**
(43) Date of publication of application: **27.11.2024**
(21) Application number: 24205490.6
(22) Date of filing: 14.02.2019
(51) Int. Cl.: A61K 45/06

(54) **COMPOSITIONS FOR THE TREATMENT OF NON-ALCOHOLIC FATTY LIVER DISEASE AND NON-ALCOHOLIC STEATOHEPATITIS**

(30) Priority: 14.02.2018 US 201862630361 P
(62) Divisional of application: 19755124.5
(71) Applicant: Lumos Pharma, Inc., Austin, TX 78756 (US)
(72) Inventor: THORNER, Michael, Oliver, Charlottesville, 22903 (US); SMITH, Roy, G., Houston, 77005 (US)
(74) Representative: Plasseraud IP

(57) **Abstract**

Described herein is a new method for treating diseases such as non-alcoholic fatty liver disease and non-alcoholic steatohepatitis with a growth hormone secretagogue alone or in combination with a drug selected from a dipeptidyl peptidase-4 antagonist, a glucagon-like peptide receptor agonist, a thiazolidinedione, a sodium glucose transport protein 2 antagonist, and metformin. Compositions relating to the same are also provided.

## Description

### FIELD OF THE INVENTION

The present invention relates to a new method for treating non-alcoholic fatty liver disease and non-alcoholic steatohepatitis with a growth hormone secretagogue or a combination of a growth hormone secretagogue and a drug selected from: a dipeptidyl peptidase-4 antagonist, a glucagon-like peptide receptor agonist, a thiazolidinedione, a sodium glucose transport protein 2 antagonist, metformin and vitamin E.

All publications, patents, patent applications, and other references cited in this application are incorporated herein by reference in their entirety for all purposes and to the same extent as if each individual publication, patent, patent application or other reference was specifically and individually indicated to be incorporated by reference in its entirety for all purposes. Citation of a reference herein shall not be construed as an admission that such is prior art to the present invention.

### BACKGROUND OF THE INVENTION

Non-alcoholic fatty liver disease (NAFLD) is the most common liver disease in the world. It is a condition in which excess fat is stored in the liver. This condition is not caused by heavy alcohol use (which would be alcoholic liver disease). NAFLD, leading to hepatic inflammation, fibrosis, and hepatocellular carcinoma, has become a major health problem and is associated with the increasing prevalence of obesity, insulin resistance, type 2 diabetes, and metabolic disease. The incidence in the U.S. population is estimated to be 25-30% and increasing. It is also estimated that about 20% of the people having NAFLD also have non-alcoholic steatohepatitis (NASH), which can lead to complications such as cirrhosis and liver cancer. The treatment of NAFLD and/or NASH is seen as another way to treat obesity, insulin resistance, type 2 diabetes, and metabolic disease.

Currently there are no effective treatments for either NAFLD or NASH. Thus, it is desirable to develop methods of treating these diseases.

### SUMMARY OF THE INVENTION

In an aspect, the present invention provides a novel method of treating non-alcoholic fatty liver disease with a growth hormone secretagogue (GHS).

In an aspect, the present invention provides a novel method of treating NAFLD with the combination of a GHS and a drug selected from: a dipeptidyl peptidase-4 (DPP4) antagonist, a glucagon-like peptide (GLP-1) receptor agonist, a thiazolidinedione, a sodium glucose transport protein 2 (SGLT2) antagonist, metformin and vitamin E.

In another aspect, the present invention provides a novel method of treating non-alcoholic steatohepatits with a growth hormone secretagogue.

In an aspect, the present invention provides a novel method of treating NASH with the combination of a GHS and a drug selected from: a dipeptidyl peptidase-4 antagonist, a glucagon-like peptide receptor agonist, a thiazolidinedione, a sodium glucose transport protein 2 antagonist, metformin and vitamin E.

### DETAILED DESCRIPTION OF THE INVENTION

The inventors discovered that growth hormone secretagogues (e.g. ibutamoren) alone or in combination with a drug selected from: a dipeptidyl peptidase-4 antagonist, a glucagon-like peptide receptor agonist, a thiazolidinedione, a sodium glucose transport protein 2 antagonist, metformin and vitamin E is expected to treat diseases such as NAFLD and NASH.

For example, the inventors discovered that ibutamoren using a novel therapeutic approach which combines the growth hormone secretagogue ibutamoren with the DPP4 antagonist Januvia^{™} is useful for treating NAFLD and NASH. Both ibutamoren and Januvia^{™} are orally active and their safety profiles are well established. The inventors believe the combination formulated, for example, as a single pill takes advantage of the property of ibutamoren to normalize GH, and Januvia^{™} which enhances glucose stimulated insulin release. Based on their different mechanisms of action, the inventors believe the combination of both will prove synergistic, or at least additive, for treating/preventing NAFLD.

The structure of ibutamoren, also referred to herein as ibutamoren mesylate, is shown below:

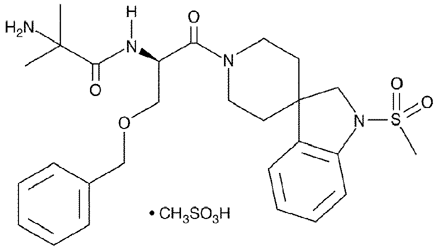

Ibutamoren is commercially available from vendors such as, for example, Sigma Aldrich and Caymen Chemical.

### NAFLD and growth hormone (GH)

NAFLD leading to hepatic inflammation, fibrosis and hepatocellular carcinoma has become a major health problem and is associated with the increasing prevalence of obesity, insulin resistance, type 2 diabetes and metabolic disease. A cross-sectional study of 7,146 individuals revealed that NAFLD is associated with low circulating growth hormone (GH) (Xu, Xu et al. 2012)). Adults with GH deficiency have low levels of insulin-like growth factor (IGF-1) and IGF binding protein-3 BP3 (IGFBP3) and are insulin resistant. Both IGF-1 and IGFBP3 are positively regulated by GH

Studies in hypophysectomized rats show that GH is critically important for regulating expression of the LDL receptor and levels of circulating lipoproteins (Rudling, Norstedt et al. 1992). Besides increasing IGF-1 and IGFBP3, GH treatment controls the activity of key enzymes involved in biosynthesis of cholesterol and bile acids. In addition, GH controls the expression of genes that enhance hydrolysis of triglycerides (TG), lowers storage of TG as well as increasing synthesis of diacylglycerol (Zhao, Cowley et al. 2011).

The binding of GH to the GH receptor (GHR) in the liver activates the transcription factor STATS. Liver selective ablation of GHR or STATS in mice results in hepatosteatosis, insulin resistance, glucose intolerance, increased triglyceride synthesis and lower efflux (Fan, Menon et al. 2009, Baik, Yu et al. 2011, Liu, Cordoba-Chacon et al. 2016). GH-STAT5 also regulates bile acid synthesis and metabolism. These properties lead to the conclusion that restoring GH to normal levels is a potential therapeutic approach to NAFLD.

GH controls the local production of cortisol by regulating 11□-hydroxysteroid dehydrogenase type-1 (HSD1), the enzyme responsible for local conversion of cortisone to the active glucocorticoid cortisol; cortisol regulates gluconeogenesis and fat deposition. HSD1 is expressed in the liver, adipose tissue and the brain. Overproduction of HSD1 in the liver increases cortisol-induced gluconeogenesis by increasing expression of the rate-limiting gluconeogenic enzyme phosphoenol-pyruvate carboxykinase. In addition, HSD1 overexpression in omental fat stimulates adipogenesis that potentially causes central obesity. It follows that inhibiting HSD1 activity to reduce local cortisol production is a potential approach for preventing and treating type 2 diabetes, obesity, age-related cognitive dysfunction and NAFLD. Inhibitors of HSD1 improve insulin sensitivity and ameliorate hepatic steatosis in db/db mice (Yuan, Li et al. 2016). Impaired GH production increases HSD1 expression and GH-deficient patients exhibit a high ratio of cortisol/cortisone that can be reversed by treating with low-dose GH.

Genetic studies show an association of NAFLD with a polymorphism in the gene encoding patatin-like phospholipase domain-containing protein 3 (PNPLA3). Although the mechanism remains obscure the variant PNPLA3-148M is associated with the full spectrum of NAFLD lesions (Boursier and Diehl 2015). Studies in obese Hispanic children expressing the genetic variant and mice expressing human PNPLA3-148M implicate dependence on intake of high carbohydrate rather than a high fat diet for development of NAFLD (Davis, Le et al. 2010, Boursier and Diehl 2015, Smagris, BasuRay et al. 2015). Recent evidence indicates that PNPLA3 is cleared by a mechanism involving ubiquitinylation, but PNPLA3-148M is resistant resulting in accumulation of the variant protein on lipid droplets (BasuRay, Smagris et al. 2017). Based on mouse studies (Smagris, BasuRay et al. 2015), increasing or decreasing PNPLA3 in subjects with two WT alleles should have little impact on steatosis, whereas increasing PNPLA3-148M would exacerbate steatosis. Since GH increases expression of wild-type PNPLA3-WT (Zhao, Cowley et al. 2011), GH therapy should prove effective in treating PNPLA3-148M heterozygotes.

### Role of insulin and growth hormone in steatosis of the liver

Obesity and type 2 Diabetes Mellitus are associated with insulin resistance. The resistance is primarily mediated at the skeletal muscle and the ability of insulin to suppress gluconeogenesis in the liver. The suppression of hepatic gluconeogenesis depends on the reduction of free fatty acids from adipose tissue (Bergman and Iyer 2017). Thus, the resistance is purely extrahepatic in skeletal muscle and adipose tissue, yet the liver retains its sensitivity to insulin to stimulate lipogenesis - this has been referred to as "selective insulin resistance" see (Titchenell, Quinn et al. 2016).

Treatment of diabetes mellitus is a balance on the one hand to regulate and control hyperglycemia while at the same time not increasing hepatic lipogenesis. Thus, increasing insulin levels by exogenous insulin or with sulfonylurea drugs does not play a part in treating NAFLD. However, GLP-1 agonists, or DPP4 antagonists which prolong the activity of endogenous GLP-1 are preferable because they enhance insulin release in response to glucose and lower blood glucose reducing the ability to enhance lipogenesis in the liver. Since it is now established that hepatic gluconeogenesis is inhibited by the effects of insulin on adipocytes to inhibit lipolysis, and that this is regulated by first phase insulin secretion, DPP4 inhibitors are ideal therapies to inhibit this process (Mest and Mentlein 2005).

Obesity and type 2 diabetes mellitus are associated with suppressed GH secretion. Insulin and GH are secreted in a pulsatile fashion and the two are tightly regulated. For example, when insulin secretion increases there is a rapid suppression of IGFBP-1 which then results in an increase in free IGF-I which feeds back to inhibit GH secretion. Following a meal, insulin levels increase to facilitate the transport of glucose into cells and to enhance storage of energy as fat. The liver is central to the integration of metabolism and stores glycogen and fat for use during times of enhanced energy utilization, e.g. exercise or starvation (Cahill 1971). After 12 to 14 hours of starvation glycogen stores from the liver are depleted and the body then turns to fat for energy. In addition to fat being stored in the liver it is also stored in white adipose tissue (primarily subcutaneous fat), which is mobilized as needed. The two hormones, which are primary in regulating the deposition of fat in liver and adipose tissue, and later its mobilization, are insulin and GH. Thus, as the time following a meal increases, insulin levels decline, and GH levels increase. On feeding, insulin rises, and GH is suppressed. The regulation of insulin and GH is complex, but both these hormones are modulated by two other hormones produced in the gastrointestinal tract, GLP-1 for insulin and ghrelin for GH. Both GLP-1 and Ghrelin act in an analogous fashion to augment the normal amplitude of insulin and GH pulses respectively. It is critical that insulin and GH are secreted at the appropriate time, usually in a reciprocal fashion, i.e. when insulin levels are high GH levels are suppressed.

### Restoration of normal profile of endogenous GH by daily oral administration of ibutamoren

Because endogenous GH is released by the anterior pituitary gland in pulses throughout the day, simply injecting recombinant GH (rhGH) does not restore the physiological profile of GH release. In GH deficient subjects administering low doses of GH improves insulin sensitivity, however high levels of GH produce insulin resistance; hence, selecting an appropriate therapeutic dose is challenging. Endogenous GH release is subject to regulatory feedback mechanisms, however administering exogenous rhGH bypasses GH negative feedback pathways. By contrast, administration of the GH-secretagogue ibutamoren enhances the amplitude of pulsatile release of endogenous GH and normalizes GH because the stimulatory effects of ibutamoren on GH pulsatility are subject to natural inhibitory feedback mediated by IGF-1; hence, hyperstimulation of the GH/IGF-1 axis is avoided (Smith, Van der Ploeg et al. 1997). It follows that ibutamoren, by recapitulating normal GH physiology is ideal for increasing insulin sensitivity in the treatment/prevention of NAFLD.

### Limitations of treating NAFLD with GLP-1 analogs or DPP4 inhibitors

GLP-1 receptor agonists, or inhibitors of the enzyme DPP4 that degrades GLP-1, enhance glucose sensitivity of pancreatic □-cells. Although the amplitude of pulsatile insulin release in response to glucose is augmented, there is not general agreement that insulin sensitivity is improved (Tominaga, Ikezawa et al. 1996, Ahren, Larsson et al. 1997). Since an important aspect of NAFLD is its association with insulin resistance, it is unlikely that targeting the GLP-1 pathway alone will have sufficient therapeutic benefit; indeed, this is supported by results reported in clinical studies. For example, Type 2 diabetic patients on metformin and/or a sulphonylurea agent were allocated for 12 week treatment with the GLP-1 receptor agonist liraglutide and/or the DPP4 inhibitor sitagliptin (Januvia^{™}); neither treatment reduced hepatic steatosis or fibrosis (Smits, Tonneijck et al. 2016). In a 24 week study with 50 NAFLD patients it was concluded that sitagliptin was no better than placebo in reducing liver fat (Cui, Philo et al. 2016). Another study with 12 subjects treated for 24 weeks with sitagliptin showed no improvement in fibrosis (Joy, McKenzie et al. 2017). The inventors suggest that a limitation of targeting the GLP-1 pathway alone for treating NAFLD is that it does not adequately relieve insulin resistance associated with NAFLD.

### Proposed treatment of NAFLD with a combination of ibutamoren and Januvia

The ideal treatment of NAFLD would restore deficient GH secretion which will then restore bile acid secretion and hepatic lipid metabolism and enhance appropriately timed insulin secretion. The inventors believe this could be achieved with a combination of, for example, ibutamoren and Januvia^{™}, with ibutamoren mimicking ghrelin, and Januvia^{™} enhancing endogenous GLP-1 by blocking DPP4 which normally breaks down GLP-1. Ibutamoren has distinct advantages over ghrelin because besides not being orally active, ghrelin also inhibits insulin release from pancreatic □-cells. By direct contrast, ibutamoren does not suppress glucose stimulated insulin secretion; therefore, ibutamoren does not negate the stimulatory effects of GLP-1 on insulin release. Thus, a pill containing the combination of ibutamoren and a DPP4 inhibitor such as Januvia^{™} would have the properties necessary for treating/preventing NAFLD.

Animal studies demonstrate that GH is an important regulator of hepatic fat metabolism. Fat accumulation in visceral fat and in the liver increase with aging which is associated with a progressive decline of GH secretion by 50% every 7-10 years from mid puberty such that elderly people have similar GH levels to those found in GH deficient young adults. GH deficient adults have an increased incidence of non-alcoholic fatty liver disease (NAFLD) and steatohepatitis. GH replacement therapy reverses this process. It is hypothesized that a GH secretagogue, such as ibutamoren, will restore pulsatile GH secretion and lower visceral fat; this is based on the observation that endogenous GH secretion correlates negatively with the amount of visceral fat and with liver fat accumulation (NAFLD) and that low dose GH reverses this process. HIV lipodystrophy is associated with both increased visceral fat accumulation and steatohepatitis. It has been treated with both supraphysiological rhGH injections and with tesamorelin (a long acting GHRH analog). Since ibutamoren has been demonstrated to enhance GH secretion, increase serum IGF-1 and increase lean body mass in obese individuals this hypothesis is supported. Ibutamoren is proposed as treatment for NAFLD and steatohepatitis due to NAFLD. The combination with various agents which improve insulin sensitivity is proposed to mitigate the mild diabetogenic action of the enhanced GH secretion induced by ibutamoren.

### Vitamin E

Oxidative stress has been implicated to have an important role in the progression of NASH. Vitamin E is well known as a free radical scavenger, and has been prescribed for the treatment of NASH. Vitamin E treatment for 1 year reduced serum transaminase activities as well as TGF-β₁ in adult NASH patients who were refractory to dietary intervention. In pioglitazone versus vitamin E versus Placebo for the Treatment of Nondiabetic Patients with Nonalcoholic Steatohepatitis (PIVENS) trial, vitamin E (800 mg/day) is superior to placebo for the improvements of NASH histology in adults NASH without diabetes and cirrhosis.

According to a random-effects model analysis of the five studies, vitamin E significantly reduced serum hepatobiliary enzymes, hepatic steatosis, inflammation, and hepatocellular ballooning compared with the control group. In those studies, however, fibrosis improvement was not confirmed.

In Japan, long-term vitamin E treatments (300 mg/day) for more than 2 years can ameliorate hepatic fibrosis in NASH patients, especially in those whose serum transaminase activities and insulin resistance can be improved. This result has suggested that metabolic factors should be controlled even when vitamin E is administrated.

Although vitamin E is now recommended only for biopsy-proven NASH patients without diabetes on the basis of PIVENS trial, it is associated with histological improvement regardless of diabetic status. However, the primary concern regarding vitamin E for NASH treatment has been the potential for toxicity with long-term or high-dose use. Vitamin E treatment may increase all-cause mortality, prostatic cancer (SELECT trial), and hemorrhagic stroke, although several conflicting results exist. When vitamin E is administrated for NASH, treatment with lower dose (300-400 mg/day rather than 800 mg) of its agent should be considered.

### Certain Embodiments of the Invention

In an aspect, the present invention provides a novel method of treating non-alcoholic fatty liver disease (NAFLD) or non-alcoholic steatohepatitis (NASH), comprising: administering to a patient in need thereof a therapeutically effective amount of a growth hormone secretagogue (GHS).

In another aspect, the novel method further comprises: administering a therapeutically effective amount of a second drug selected from: a dipeptidyl peptidase-4 (DPP4) antagonist, a glucagon-like peptide (GLP-1) receptor agonist, a thiazolidinedione, a sodium glucose transport protein 2 (SGLT2) antagonist, metformin and vitamin E.

Patient refers to a human patient, either child or adult.

In another aspect, the disease is NAFLD.

In another aspect, the disease is NASH.

In another aspect, the GHS is ibutamoren (iibutamoren mesylate).

In another aspect, 10-50 mg of ibutamoren is administered, for example, once daily. In one embodiment, 25-50 mg of ibutamoren is administered once daily. Other examples of the amount of ibutamoren administered include 10, 15, 20, 25, 30, 35, 40, 45, and 50 mg. In another aspect, ibutamoren is administered orally.

In another aspect, the second drug is a DPP4 antagonist.

In another aspect, the DPP4 antagonist is selected from:
sitagliptin (tradename Januvia^{®}, typically dosed at 25-100 mg/day, orally);
vildagliptin (tradename Salvus^{®}, typically dosed at 50 mg twice/day, orally);
saxagliptin (tradename Onglyza^{®}, typically dosed at 2.5 or 5 mg/day, orally);
linagliptin (tradename Tradjenta^{®}, typically dosed at 5 mg/day, orally); and,
alogliptin (tradename Nesina^{®}, typically dosed at 6.25, 12.5, and 25 mg/day, orally).

In another aspect, the second drug is a GLP-1 receptor agonist.

In another aspect, the GLP-1 receptor agonist is selected from:
exenatide (tradenames Byetta^{®} and Bydureon^{®}, typically dosed at 2 mg once/week by injection);
liraglutide (tradenames Victoza^{®} and Saxenda^{®}, typically dosed at 1.2 mg/day by injection);
lixisenatide (tradename Adylxin^{®}, typically dosed at 20 µg/day by injection);
albiglutide (tradename Tanzeum^{®}, typically dosed at 30 mg, once/week by injection);
dulaglutide (tradename Trulicity^{®}, typically dosed at 0.75-1.5 mg once/week by injection); and,
semaglutide (tradename Ozempic^{®}, typically dosed at 0.5-1 mg once/week by injection).

In another aspect, the second drug is a thiazolidinedione (TZD). Thiazolidinediones (also called glitazones) are a class of drugs that have hypoglycemic action (e.g., antihyperglycemic and/or antidiabetic).

In another aspect, the TZD is selected from:
pioglitazone (tradename Actos^{®}, typically dosed at 15 mg, 30 mg, or 45 mg/day, orally); and,
rosiglitazone (tradename Avandia^{®}, typically dosed at 4 mg (2 mg + 2 mg or 4 mg in one dose) or 8 mg/day, orally.

In another aspect, the second drug is a sodium glucose transport protein 2 (SGLT2) antagonist.

In another aspect, the SGLT2 antagonist is selected from:
empagliflozin (tradename Jardiance^{®}, typically dosed at 5, 10, or 12.5 mg/day, orally, depending on whether dosed alone in combination with metformin); and,
dapagliflozin (tradename Farxiga^{®}, typically dosed at 2.5, 5, or 10 mg/day, orally, depending on whether dosed alone in combination with metformin).

In another aspect, the second drug is metformin. Metformin is available in a wide variety of dosages including immediate release tablets of 500, 850, and 1000 mg and extended-release tables of 500, 750, and 1000 mg. Metformin is typically dosed at 1500, 2000, 2500, to 2550 mg/day, orally.

In another aspect, in the method of treating, the GHS is ibutamoren and the second drug is selected from:
(i.) sitagliptin;
(ii.) vildagliptin;
(iii.) saxagliptin;
(iv.) linagliptin;
(v.) alogliptin;
(vi.) pioglitazone;
(vii.) rosiglitazone;
(viii.) empagliflozin;
(ix.) dapagliflozin; and,
(x.) metformin.

In another aspect, in the method of treating, the GHS is ibutamoren and the second drug is sitagliptin.

In another aspect, in the method of treating, the GHS is ibutamoren and the second drug is pioglitazone.

In another aspect, in the method of treating, the GHS is ibutamoren and the second drug is metformin.

In another aspect, the present invention provides a novel method of treating treating non-alcoholic fatty liver disease (NAFLD) or non-alcoholic steatohepatitis (NASH), comprising: administering to a patient in need thereof:
(i.) a therapeutically effective amount of a growth hormone secretagogue (GHS);
(ii.) a therapeutically effective amount of a second drug selected from: a dipeptidyl peptidase-4 (DPP4) antagonist, a glucagon-like peptide (GLP-1) receptor agonist, a thiazolidinedione, and, a sodium glucose transport protein 2 (SGLT2) antagonist; and,
(iii.) a therapeutically effective amount of a third drug, which is metformin.

In another aspect, the second drug is selected from a dipeptidyl peptidase-4 (DPP4) antagonist, a thiazolidinedione, and, a sodium glucose transport protein 2 (SGLT2) antagonist.

In another aspect, the second drug is a dipeptidyl peptidase-4 (DPP4) antagonist.

In another aspect, the second drug is a thiazolidinedione.

In another aspect, the second drug is a sodium glucose transport protein 2 (SGLT2) antagonist.

In another aspect, in the method of treating, the GHS is ibutamoren, the second drug is sitagliptin, and the third drug is metformin.

In another aspect, in the method of treating, the GHS is ibutamoren, the second drug is pioglitazone, and the third drug is metformin.

The timing of the dosage of the first (i.e., the GHS) and second drugs (or first, second, and third drugs) depends on their independent dosage regimen. Examples of dosage timing include:
(i.) Simultaneous dosing, single formulation. This can be achieved by co-formulating the drugs (two or three) into a single formulation (e.g., an oral dosage) and then administering the single formulation.
(ii.) Simultaneous dosing, different formulations. This can be achieved by independently administering the drugs at approximately the same time (e.g., different oral dosages, oral/injected dosages, or injected dosages).
(iii.) Simultaneous dosing + additional dosing. For drugs that are administered with overlapping timing (e.g., in the morning, but only one in the evening), one of regimens (i.) or (ii.) can be used followed by additional dosing of drugs.
(iv.) Non-simultaneous dosing. For drugs that are administered with different timing (e.g., daily oral dosage versus weekly injection), the drugs can be administered in accordance with their individual protocols.

One of the potential benefits of the present invention is the potential to administer the first and second (or first, second, and third) drugs simulataneously. For example, if the second (or second and third) drug can be administered orally, then the first and second (or second and third) drugs can be formulated into a single, oral dosage (e.g., pill, tablet, capsule, powder, liquid suspension, etc.).

In another aspect, the present invention provides a novel drug composition, comprising:
(i.) a therapeutically effective amount of a growth hormone secretagogue (GHS);
(ii.) a therapeutically effective amount of a second drug selected from: dipeptidyl peptidase-4 (DPP4) antagonist, a thiazolidinedione, a sodium glucose transport protein 2 (SGLT2) antagonist, and metformin; and,
(iii.) a pharmaceutically acceptable carrier;
wherein the composition is useful in treating NAFLD and/or NASH.

In another aspect, the composition is orally or parenterally administerable.

In another aspect, the second drug is a DPP4 antagonist.

In another aspect, the GHS is ibutamoren and the second drug is sitagliptin.

In another aspect, the second drug is a thiazolidinedione.

In another aspect, the GHS is ibutamoren and the second drug is pioglitazone.

In another aspect, the second drug is a SGLT2 antagonist.

In another aspect, the second drug is metformin.

In another aspect, the GHS is ibutamoren and the second drug is metformin.

In another aspect, the present invention provides a novel triple drug composition, comprising:
(i.) a therapeutically effective amount of a growth hormone secretagogue (GHS); and,
(ii.) a therapeutically effective amount of a second drug selected from: dipeptidyl peptidase-4 (DPP4) antagonist, a thiazolidinedione, and a sodium glucose transport protein 2 (SGLT2) antagonist;
(iii.) a therapeutically effective amount of a third drug, which is metformin;
(iv.) a pharmaceutically acceptable carrier;
wherein the composition is useful in treating NAFLD and/or NASH.

In another aspect, the triple drug composition is orally or parentally administerable.

In another aspect, in the triple combination, the second drug is a DPP4 antagonist.

In another aspect, in the triple combination, the GHS is ibutamoren and the second drug is sitagliptin.

In another aspect, in the triple combination, the second drug is a thiazolidinedione.

In another aspect, in the triple combination, the GHS is ibutamoren and the second drug is pioglitazone.

In another aspect, in the triple combination, the second drug is a SGLT2 antagonist.

In another aspect, the present invention provides a novel packaging kit, comprising:
(i.) at least one first compartment, comprising: a therapeutically effective amount of a growth hormone secretagogue (GHS) and a pharmaceutically acceptable carrier;
(ii.) at least one second compartment, comprising: a therapeutically effective amount of a second drug selected from: dipeptidyl peptidase-4 (DPP4) antagonist, a thiazolidinedione, a sodium glucose transport protein 2 (SGLT2) antagonist, and metformin and a pharmaceutically acceptable carrier.

In another aspect, the present invention provides a novel packaging kit, comprising:
(i.) at least one first compartment, comprising: a therapeutically effective amount of a growth hormone secretagogue (GHS) and a pharmaceutically acceptable carrier;
(ii.) at least one second compartment, comprising: a therapeutically effective amount of a second drug selected from: dipeptidyl peptidase-4 (DPP4) antagonist, a thiazolidinedione, and a sodium glucose transport protein 2 (SGLT2) antagonist and a pharmaceutically acceptable carrier;
(iii.) at least one third compartment, comprising: a therapeutically effective amount of a third drug, which is metformin and a pharmaceutically acceptable carrier.

In another aspect, the present invention provides the use of the first and second drugs for the manufacture of a medicament for the treatment of an indication recited herein.

In another aspect, the present invention provides the use of the first, second, and third drugs for the manufacture of a medicament for the treatment of an indication recited herein.

In another aspect, the present invention provides a novel composition comprising the first and second drugs for use in the treatment of an indication recited herein.

In another aspect, the present invention provides a novel composition comprising the first, second, and third drugs for use in the treatment of an indication recited herein.

Most of the approved drugs recited herein have a specific pharmaceutical salt (e.g., ibutamoren is a mesylate, ibutamoren mesylate). While the approved salt is what is referenced above, other pharmaceutically acceptable salts are considered to be part of the presently claimed invention.

The present invention may be embodied in other specific forms without departing from the spirit or essential attributes thereof. This invention encompasses all combinations of aspects of the invention noted herein. It is understood that any and all embodiments of the present invention may be taken in conjunction with any other embodiment or embodiments to describe additional embodiments. It is also to be understood that each individual element of the embodiments is intended to be taken individually as its own independent embodiment. Furthermore, any element of an embodiment is meant to be combined with any and all other elements from any embodiment to describe an additional embodiment.

### Definitions

"Treating" or "treatment" covers the treatment of a disease-state in a mammal, and includes: (a) preventing the disease-state from occurring in a mammal, in particular, when such mammal is predisposed to the disease-state but has not yet been diagnosed as having it; (b) inhibiting the disease-state, e.g., arresting it development; and/or (c) relieving the disease-state, e.g., causing regression of the disease state until a desired endpoint is reached. Treating also includes the amelioration of a symptom of a disease (e.g., lessen the pain or discomfort), wherein such amelioration may or may not be directly affecting the disease (e.g., cause, transmission, expression, etc.).

"Pharmaceutically acceptable salts" refer to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include, but are not limited to, those derived from inorganic and organic acids selected from 1, 2-ethanedisulfonic, 2-acetoxybenzoic, 2-hydroxyethanesulfonic, acetic, ascorbic, benzenesulfonic, benzoic, bicarbonic, carbonic, citric, edetic, ethane disulfonic, ethane sulfonic, fumaric, glucoheptonic, gluconic, glutamic, glycolic, glycollyarsanilic, hexylresorcinic, hydrabamic, hydrobromic, hydrochloric, hydroiodide, hydroxymaleic, hydroxynaphthoic, isethionic, lactic, lactobionic, lauryl sulfonic, maleic, malic, mandelic, methanesulfonic, napsylic, nitric, oxalic, pamoic, pantothenic, phenylacetic, phosphoric, polygalacturonic, propionic, salicyclic, stearic, subacetic, succinic, sulfamic, sulfanilic, sulfuric, tannic, tartaric, and toluenesulfonic.

The pharmaceutically acceptable salts of the present invention can be synthesized from the parent compound that contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, non-aqueous media like ether, ethyl acetate, ethanol, isopropanol, or acetonitrile are useful. Lists of suitable salts are found in Remington's Pharmaceutical Sciences, 18th ed., Mack Publishing Company, Easton, PA, 1990, p 1445, the disclosure of which is hereby incorporated by reference.

"Therapeutically effective amount" includes an amount of a compound of the present invention that is effective when administered alone or in combination to treat obesity, diabetes, dyslipidemias, cardiovascular disorders, inflammatory disorders, hepatic disorders, cancers, and a combination or comorbitity thereof, or another indication listed herein. "Therapeutically effective amount" also includes an amount of the combination of compounds claimed that is effective to treat the desired indication. The combination of compounds can be a synergistic combination. Synergy, as described, for example, by Chou and Talalay, Adv. Enzyme Regul. 1984, 22:27-55, occurs when the effect of the compounds when administered in combination is greater than the additive effect of the compounds when administered alone as a single agent. In general, a synergistic effect is most clearly demonstrated at sub-optimal or lower doses of the compounds. Synergy can be in terms of lower cytotoxicity, increased effect, or some other beneficial effect of the combination compared with the individual components.

In the present invention, the compound(s) of the present invention can be administered in any convenient manner (e.g., enterally or parenterally). Examples of methods of administration include orally and transdermally. One skilled in this art is aware that the routes of administering the compounds of the present invention may vary significantly. In addition to other oral administrations, sustained and/or modified release compositions may be favored. Other acceptable routes may include injections (e.g., intravenous, intramuscular, subcutaneous, and intraperitoneal); subdermal implants; and, buccal, sublingual, topical, rectal, vaginal, and intranasal administrations. Bioerodible, non-bioerodible, biodegradable, and non-biodegradable systems of administration may also be used. Examples of oral formulations include tablets, coated tablets, hard and soft gelatin capsules, solutions, emulsions, powders, granules, and suspensions.

If a solid composition in the form of tablets is prepared, the active ingredient(s) can be mixed with a pharmaceutical vehicle, examples of which include silica, starch, lactose, magnesium stearate, and talc. The tablets can be optionally coated with sucrose or another appropriate substance or they can be treated so as to have a sustained or delayed activity and so as to release a predetermined amount of active ingredient continuously. Capsules can be obtained, for example, by mixing the active ingredient(s) with a diluent and incorporating the resulting mixture into soft or two piece hard capsules. By way of example, a syrup or elixir can contain the active ingredient(s) in conjunction with a sweetener, which is typically calorie-free, an antiseptic (e.g., methylparaben and/or propylparaben), a flavoring, and an appropriate color. Water-dispersible powders or granules, for instance, can contain the active ingredient(s) mixed with dispersants or wetting agents or with suspending agents such as polyvinylpyrrolidone, as well as with sweeteners or taste correctors. Rectal administration can be effected using suppositories, which are prepared with binders melting at the rectal temperature (e.g., cocoa butter and/or polyethylene glycols), gels or foams. Parenteral administration can be effected using aqueous suspensions, isotonic saline solutions, or injectable sterile solutions, which contain pharmacologically compatible dispersants and/or wetting agents (e.g., propylene glycol and/or polyethylene glycol). The active ingredient(s) can also be formulated as microcapsules or microspheres, optionally with one or more carriers or additives. The active ingredient(s) can also be presented in the form of a complex with a cyclodextrin, for example α-, β-, or γ-cyclodextrin, 2-hydroxypropyl-β-cyclodextrin, and/or methyl-β-cyclodextrin.

The dose of the compound of the present invention administered daily will vary on an individual basis and to some extent may be determined by the severity of the disease being treated (e.g., NAFLD or NASH). The dose of the compound of the present invention will also vary depending on the drug or drugs administered. Examples of dosages of compounds of the present invention have been provided above but may vary based on synergistic effects of a combination of two or three drugs.

The compound can be administered in a single dose or in a number of smaller doses over a period of time. The length of time during which the compound is administered varies on an individual basis and can continue until the desired results are achieved (i.e., reduction of body fat, or prevention of a gain in body fat).

The disclosure is further illustrated by the following examples, which are not to be construed as limiting this disclosure in scope or spirit to the specific procedures herein described. It is to be understood that the examples are provided to illustrate certain embodiments and that no limitation to the scope of the disclosure is intended thereby. It is to be further understood that resort may be had to various other embodiments, modifications, and equivalents thereof which may suggest themselves to those skilled in the art without departing from the spirit of the present disclosure and/or scope of the appended claims.

### EXAMPLES

### Example 1

Examples of oral compositions of the present invention are provided in the present table (only active ingredients are shown).

| **Ex. No.** | **ibutamoren (mg)** | **Sitagliptin (mg)** | **Pioglitazone (mg)** | **Metformin (mg)** |
|---|---|---|---|---|
| 1 | 25 | 50 | - | - |
| 2 | 25 | - | 15 | - |
| 3 | 25 | - | - | 500 |
| 4 | 25 | 50 | - | 500 |
| 5 | 25 | - | 15 | 500 |
| 6 | 25 | 50 | 15 | 500 |

### Example 2

Further examples of oral compositions of the present invention are provided in the present table (only active ingredients are shown):

| | |
|---|---|
| ibutamoren | 10 mg, 25 mg, 50 mg, 100 mg |
| Pioglitazone eg of thiazolidinedione | 30 mg, 45 mg |
| Metformin | 850 mg, 1000 mg, 1500 mg |
| Sitagliptin eg of dipeptidyl-4 (DPP4) antagonist | 50 mg, 100 mg, 200 mg |
| Semaglutide oral eg of GLP-1 agonist | 3 mg, 7 mg, 14 mg |
| Vitamin E | 300 mg, 400 mg, 800 mg |

### Example 3

Growth hormone is a hormone produced in the pituitary gland that helps regulate metabolism and growth. Individuals with obesity, on average, secrete less growth hormone than individuals without obesity. There are data to suggest that growth hormone may help to reduce the amount of fat in the liver, and may also reduce inflammation in the liver, both of which would be helpful to individuals with NAFLD. The purpose of this proposed study is to investigate whether treatment with ibutamoren, also known as ibutamoren mesylate, which is a growth hormone secretagogue, will decrease liver fat and improve liver inflammation and scarring in obese individuals with NAFLD.

| **Condition or disease** | **Intervention/treatment** | **Phase** |
|---|---|---|
| Non-Alcoholic Fatty Liver Disease | Drug: ibutamoren Drug: Identical Placebo | Phase 2 |
| Obesity | | |
| Obesity, Abdominal | | |
| Liver Fat | | |
| Fatty Liver | | |

| | |
|---|---|
| Study Type : | Interventional (Clinical Trial) |
| Estimated Enrollment: | 76 participants |
| Allocation: | Randomized |
| Intervention Model: | Parallel Assignment |
| Intervention Model Description: | Randomized, double-blind, placebo controlled phase for first 12 months, followed by open-label phase for 6 months during which all participants receive active medication (ibutamoren) |
| Masking: | Quadruple (Participant, Care Provider, Investigator, Outcomes Assessor) |
| Primary Purpose: | Treatment |
| Official Title: | Growth Hormone Secretagogue to Improve Nonalcoholic Fatty Liver Disease and Associated Cardiovascular Risk |

### Arms and Interventions

| **Arm** | **Intervention/treatment** |
|---|---|
| Experimental: Ibutamoren (LUM-201) 25 mg tablet [Lumos Pharma] | Drug: Ibutamoren |
| | Ibutamoren 25 mg po daily |
| | Other Name: ibutamoren; LUM-210, |
| | Oratrope^{™} |
| Placebo Comparator: Placebo identical placebo given orally daily | Drug: Identical Placebo |
| | Placebo tablet daily |

### Outcome Measures

Primary Outcome Measures :
- Liver Fat Content [Time Frame: change from baseline to 12 months]
- Liver Fat Content as measured by hydrogen-magnetic resonance spectroscopy

Secondary Outcome Measures :
- NAFLD Activity Score [Time Frame: change from baseline to 12 months]
- NAFLD Activity Score (NAS, scored between 0-8) from liver biopsy
- Post-prandial hepatic de novo lipogenesis [Time Frame: change from baseline to 12 months]
- hepatic de novo lipogenesis as measured by stable isotope methods
- Coronary plaque volume [Time Frame: change from baseline to 12 months]
   Volume of calcified and noncalcified plaque determine by coronary computed tomography angiography (CCTA)
- Non-high density lipoprotein (Non-HDL) Cholesterol [Time Frame: change from baseline to 12 months]
- C-reactive protein [Time Frame: change from baseline to 12 months]
- Fibrosis Score [Time Frame: change from baseline to 12 months]
- fibrosis score from liver biopsy

### Eligibility Criteria

| | |
|---|---|
| Ages Eligible for Study: | 18 Years to 70 Years (Adult, Older Adult) |
| Sexes Eligible for Study: | All |
| Accepts Healthy Volunteers: | No |

### Criteria

Inclusion Criteria:
- Men and women 18-70yo
- Body mass index (BMI) ≥ 30kg/m2
- Hepatic steatosis as demonstrated by either a) Grade 1+ steatosis on a liver biopsy performed within 12 months of the baseline visit, without >10% reduction in body weight or addition of medications to treat fatty liver, or b) liver fat fraction ≥5% on hydrogen-magnetic resonance spectroscopy (1H-MRS)
- Hepatitis C antibody and Hepatitis B surface antigen negative
- For females ≥50yo, negative mammogram within 1 year of baseline
- If use of vitamin E ≥400 international units daily, stable dose for ≥6 mos

Exclusion Criteria:
- Heavy alcohol use defined as consumption of > 20 grams daily for women or > 30 grans daily for men for at least 3 consecutive months over the past 5 years assessed using the Lifetime Drinking History Questionnaire
- Known diagnosis of diabetes, use of any anti-diabetic medications (including thiazolidinediones or metformin), fasting glucose >126mg/dL, or hemoglobin A1c (HbA1c) ≥7%
- Use of any specific pharmacological treatments for NAFLD/nonalcoholic steatohepatitis except vitamin E
- Known cirrhosis, Child-Pugh score ≥7, stage 4 fibrosis on biopsy, or clinical evidence of cirrhosis or portal hypertension on imaging or exam. If a subject is not known to be cirrhotic at screen but is found to be cirrhotic based on the results of liver biopsy at baseline, this subject will be referred to a hepatologist for clinical care and will be excluded from further participation in the study.
- Chronic systemic corticosteroid use in the ≤6 months prior to the baseline visit
- Chronic use of Actigall, methotrexate, amiodarone, or tamoxifen
- Known diagnosis of alpha-1 antitrypsin deficiency, Wilson's disease, hemochromatosis, or autoimmune hepatitis
- Use of growth hormone or growth hormone releasing hormone or GH secreatgague within the past 1 year
- Change in lipid lowering or anti-hypertensive regimen within 2 months of screening
- Hemoglobin < 10.0 g/dL or Creatinine >1.5mg/dL
- Active malignancy
- For men, history of prostate cancer or evidence of prostate malignancy by prostate specific antigen (PSA) > 5 ng/mL
- Severe chronic illness judged by the investigator to present a contraindication to participation
- History of hypopituitarism, head irradiation or any other condition known to affect the GH axis
- Use of physiologic testosterone (men) or estrogen or progesterone (women) unless stable use for a year or more prior to study entry
- Routine magnetic resonance imaging (MRI) exclusion criteria such as the presence of a pacemaker or cerebral aneurysm clip
- Weight loss surgery within 2 years before baseline. Weight loss surgery more than 2 years prior to baseline visit is permissible as long as no active weight loss (<10% decrease in weight over past 6 months)
- For women, positive urine pregnancy test (hCG), trying to achieve pregnancy, or breastfeeding
- Known hypersensitivity to ibutamoren
- Contraindication to receiving beta-blocker or nitroglycerin (which are part of the coronary angiography)
- Significant radiation exposure, including any history of radiation therapy, or any of the following in the 12 months prior to randomization: a) more than 2 percutaneous coronary interventions; b) more than 2 myocardial perfusion studies; 3) more than 2 computed tomography angiograms
- Active consideration for a procedure or treatment that involves significant radiation exposure as defined above in the 12 months following randomization
- Not willing or able to adhere to dose schedules and required procedures per protocol
- Judged by the investigator to be inappropriate for the study for other reasons not detailed above.

### Example 4

The purpose of this proposed study is to investigate whether treatment with ibutamoren, also known as ibutamoren mesylate, which is a growth hormone secretagogue, in combination with a second active ingredient, will decrease liver fat and improve liver inflammation and scarring in obese individuals with NAFLD.

| **Condition or disease** | **Intervention/treatment** | **Phase** |
|---|---|---|
| Non-Alcoholic Fatty Liver Disease | Drug: ibutamoren | Phase 2 |
| Obesity | Drug: Identical Placebo | |
| Obesity, Abdominal | Drug: Pioglitazone | |
| | Drug: Identical Placebo | |
| Liver Fat | | |
| Fatty Liver | | |

### Arms and Interventions

| **Arm** | **Intervention/treatment** |
|---|---|
| Experimental: Ibutamoren (LUM-201) 25 mg tablet [Lumos Pharma] | Drug: Ibutamoren |
| | Ibutamoren 25 mg po daily |
| | Other Name: ibutamoren; LUM-210, |
| | Oratrope^{™} |
| Experimental: Ibutamoren (LUM-201) 25 mg plus Pioglitazone 30 mg for two months followed by 45 mg for remaining 10 months | Drug: Ibutamoren (LUM-201) plus |
| | Pioglitazone 30 mg or 45 mg |
| Placebo Comparator: Placebo identical placebo given orally daily | Drug: Identical Placebo |
| | Placebo tablet daily |

### Outcome Measures

Primary Outcome Measures :
- Liver Fat Content [Time Frame: change from baseline to 12 months]
- Liver Fat Content as measured by hydrogen-magnetic resonance spectroscopy

Secondary Outcome Measures :
- NAFLD Activity Score [Time Frame: change from baseline to 12 months]
   NAFLD Activity Score (NAS, scored between 0-8) from liver biopsy
- Post-prandial hepatic de novo lipogenesis [Time Frame: change from baseline to 12 months]
   hepatic de novo lipogenesis as measured by stable isotope methods
   Coronary plaque volume [Time Frame: change from baseline to 12 months]
   Volume of calcified and noncalcified plaque determine by coronary computed tomography angiography (CCTA)
- Non-high density lipoprotein (Non-HDL) Cholesterol [Time Frame: change from baseline to 12 months]
- C-reactive protein [Time Frame: change from baseline to 12 months]
   Fibrosis Score [Time Frame: change from baseline to 12 months]
   fibrosis score from liver biopsy

### Eligibility Criteria

| | |
|---|---|
| Ages Eligible for Study: | 18 Years to 70 Years (Adult, Older Adult) |
| Sexes Eligible for Study: | All |
| Accepts Healthy Volunteers: | No |

### Criteria

Inclusion Criteria:
- Men and women 18-70yo
- Body mass index (BMI) ≥ 30kg/m2
- Hepatic steatosis as demonstrated by either a) Grade 1+ steatosis on a liver biopsy performed within 12 months of the baseline visit, without >10% reduction in body weight or addition of medications to treat fatty liver, or b) liver fat fraction ≥5% on hydrogen-magnetic resonance spectroscopy (1H-MRS)
- Hepatitis C antibody and Hepatitis B surface antigen negative
- For females ≥50yo, negative mammogram within 1 year of baseline
   If use of vitamin E ≥400 international units daily, stable dose for ≥6 mos

Exclusion Criteria:
- Heavy alcohol use defined as consumption of > 20 grams daily for women or > 30 grans daily for men for at least 3 consecutive months over the past 5 years assessed using the Lifetime Drinking History Questionnaire
- Known diagnosis of diabetes, use of any anti-diabetic medications (including thiazolidinediones or metformin), fasting glucose >126mg/dL, or hemoglobin A1c (HbA1c) ≥7%
- Use of any specific pharmacological treatments for NAFLD/nonalcoholic steatohepatitis except vitamin E
- Known cirrhosis, Child-Pugh score ≥7, stage 4 fibrosis on biopsy, or clinical evidence of cirrhosis or portal hypertension on imaging or exam. If a subject is not known to be cirrhotic at screen but is found to be cirrhotic based on the results of liver biopsy at baseline, this subject will be referred to a hepatologist for clinical care and will be excluded from further participation in the study.
- Chronic systemic corticosteroid use in the ≤6 months prior to the baseline visit
- Chronic use of Actigall, methotrexate, amiodarone, or tamoxifen
- Known diagnosis of alpha-1 antitrypsin deficiency, Wilson's disease, hemochromatosis, or autoimmune hepatitis
- Use of growth hormone or growth hormone releasing hormone or GH secreatgague within the past 1 year
- Change in lipid lowering or anti-hypertensive regimen within 2 months of screening
- Hemoglobin < 10.0 g/dL or Creatinine >1.5mg/dL
- Active malignancy
- For men, history of prostate cancer or evidence of prostate malignancy by prostate specific antigen (PSA) > 5 ng/mL
- Severe chronic illness judged by the investigator to present a contraindication to participation
- History of hypopituitarism, head irradiation or any other condition known to affect the GH axis
- Use of physiologic testosterone (men) or estrogen or progesterone (women) unless stable use for a year or more prior to study entry
- Routine magnetic resonance imaging (MRI) exclusion criteria such as the presence of a pacemaker or cerebral aneurysm clip
- Weight loss surgery within 2 years before baseline. Weight loss surgery more than 2 years prior to baseline visit is permissible as long as no active weight loss (<10% decrease in weight over past 6 months)
- For women, positive urine pregnancy test (hCG), trying to achieve pregnancy, or breastfeeding
- Taking any drug which is a strong CYP3A4 inhibitor, eg ketoconazole, some protease inhibitors (eg Ritonavir)
- Contraindication to receiving beta-blocker or nitroglycerin (which are part of the coronary angiography)
- Significant radiation exposure, including any history of radiation therapy, or any of the following in the 12 months prior to randomization: a) more than 2 percutaneous coronary interventions; b) more than 2 myocardial perfusion studies; 3) more than 2 computed tomography angiograms
- Active consideration for a procedure or treatment that involves significant radiation exposure as defined above in the 12 months following randomization
- Not willing or able to adhere to dose schedules and required procedures per protocol
- Since FDA has warned that overall, the data suggest that pioglitazone use may be linked to an increased risk of bladder cancer. Therefore, in this trial, patients with a history of or presence of bladder cancer will not be included in this study
- Judged by the investigator to be inappropriate for the study for other reasons not detailed above.

Numerous modifications and variations of the present invention are possible in light of the above teachings. It is therefore to be understood that within the scope of the appended claims, the invention may be practiced otherwise that as specifically described herein.

The present invention can be summarized by reference to the following embodiments:
**1.** A method of treating non-alcoholic fatty liver disease (NAFLD) or non-alcoholic steatohepatits (NASH), comprising: administering to a patient in need thereof a therapeutically effective amount of a growth hormone secretagogue (GHS).
**2.** The method of Embodiment 1, wherein the disease is NAFLD.
**3.** The method of Embodiment 1, wherein the disease is NASH.
**4.** The method of Embodiment 1, wherein the GHS is ibutamoren.
**5.** The method of Embodiment 4, wherein the therapeutically amount of ibutamoren is 25-50 mg/day.
**6.** A method of treating non-alcoholic fatty liver disease (NAFLD) or non-alcoholic steatohepatits (NASH), comprising the steps of administering to a patient in need thereof:
   a therapeutically effective amount of a growth hormone secretagogue (GHS); and
   a therapeutically effective amount of a second drug selected from dipeptidyl peptidase-4 (DPP4) antagonist, a glucagon-like peptide (GLP-1) receptor agonist, a thiazolidinedione, a sodium glucose transport protein 2 (SGLT2) antagonist, metformin and vitamin E.
**7.** The method of Embodiment 6, wherein the GHS is ibutamoren.
**8.** The method of Embodiment 6, wherein the therapeutically effective amount of ibutamoren is 25-50 mg/day.
**9.** The method of Embodiment 6, wherein the second drug is a DPP4 antagonist.
**10.** The method of Embodiment 6, wherein the DPP4 antagonist is sitagliptin.
**11.** The method of Embodiment 6, wherein the GHS is ibutamoren and the second drug is sitagliptin.
**12.** The method of Embodiment 6, wherein the second drug is a GLP-1 receptor agonist.
**13.** The method of Embodiment 6, wherein the second drug is a thiazolidinedione.
**14.** The method of Embodiment 6, wherein the second drug is pioglitazone.
**15.** The method of Embodiment 6, wherein the GHS is ibutamoren and the second drug is pioglitazone.
**16.** The method of Embodiment 6, wherein the second drug is a SGLT2 antagonist.
**17.** The method of Embodiment 6, wherein the second drug is metformin.
**18.** The method of Embodiment 6, wherein the GHS is ibutamoren and the second drug is metformin.
**19.** A method of treating NAFLD or NASH, comprising: administering to a patient in need thereof:
   a a therapeutically effective amount of a growth hormone secretagogue (GHS);
   b a therapeutically effective amount of a second drug selected from: a dipeptidyl peptidase-4 (DPP4) antagonist, a glucagon-like peptide (GLP-1) receptor agonist, a thiazolidinedione, and, a sodium glucose transport protein 2 (SGLT2) antagonist; and,
   c a therapeutically effective amount of a third drug, which is metformin.
**20.** The method of Embodiment 19, wherein the therapeutically effective amount of ibutamoren is 25-50 mg/day.
**21.** The method of Embodiment 19, wherein the GHS is ibutamoren and the second drug is sitagliptin.
**22.** The method of Embodiment 19, wherein the GHS is ibutamoren and the second drug is pioglitazone.
**23.** A drug composition, comprising:
   a a therapeutically effective amount of a GHS;
   b a therapeutically effective amount of a second drug selected from: a DPP4 antagonist, a thiazolidinedione, a SGLT2 antagonist, metformin and vitamin E; and,
   c a pharmaceutically acceptable carrier;
   wherein the composition is useful in treating NAFLD and/or NASH.
**24.** The drug composition of Embodiment 23, wherein the GHS is ibutamoren.
**25.** The drug composition of Embodiment 23, wherein the therapeutically effective amount of ibutamoren is 25-50 mg/day.
**26.** A drug composition, comprising:
   a a therapeutically effective amount of a GHS;
   b a therapeutically effective amount of a second drug selected from: a DPP4 antagonist, a thiazolidinedione, and a SGLT2 antagonist;
   c a therapeutically effective amount of a third drug, which is metformin; and,
   d a pharmaceutically acceptable carrier;
   wherein the composition is useful in treating NAFLD and/or NASH.
**27.** The drug composition of Embodiment 26, wherein the GHS is ibutamoren.
**28.** The drug composition of Embodiment 26, wherein the therapeutically effective amount of ibutamoren is 25-50 mg/day.

## Claims

1. A growth hormone secretagogue (GHS) for use in a method of treating non-alcoholic fatty liver disease (NAFLD) or non-alcoholic steatohepatits (NASH), comprising: administering to a patient in need thereof a therapeutically effective amount of said growth hormone secretagogue.

2. A growth hormone secretagogue (GHS) for use according to claim 1, wherein the disease is NAFLD, or NASH.

3. A growth hormone secretagogue (GHS) for use according to claim 1, wherein the GHS is ibutamoren.

4. A growth hormone secretagogue (GHS) for use according to claim 3, wherein the therapeutically amount of ibutamoren is 25-50 mg/day.

5. A composition comprising :
- a therapeutically effective amount of a growth hormone secretagogue (GHS); and
- a therapeutically effective amount of a second drug selected from dipeptidyl peptidase-4 (DPP4) antagonist, a glucagon-like peptide (GLP-1) receptor agonist, a thiazolidinedione, a sodium glucose transport protein 2 (SGLT2) antagonist, metformin and vitamin E, for use in a method of treating non-alcoholic fatty liver disease (NAFLD) or non-alcoholic steatohepatits (NASH), comprising the steps of administering said composition to a patient in need thereof.

6. A composition for use according to claim 5, wherein the GHS is ibutamoren.

7. A composition for use according to claim 5 or 6, wherein the second drug is selected from sitagliptin, pioglitazone, and metformin.

8. A composition comprising ::
a a therapeutically effective amount of a growth hormone secretagogue (GHS);
b a therapeutically effective amount of a second drug selected from: a dipeptidyl peptidase-4 (DPP4) antagonist, a glucagon-like peptide (GLP-1) receptor agonist, a thiazolidinedione, and, a sodium glucose transport protein 2 (SGLT2) antagonist; and,
c a therapeutically effective amount of a third drug, which is metformin, for use in a method of treating NAFLD or NASH, comprising administering said composition to a patient in need thereof.

9. A composition for use according to claim 8, wherein the GHS is ibutamoren.

10. A composition for use according to claim 6 or 9, wherein the therapeutically effective amount of ibutamoren is 25-50 mg/day.

11. A composition for use according to claim 8, wherein the GHS is ibutamoren and the second drug is selected from sitagliptin, and pioglitazone.

12. A drug composition, comprising:
a a therapeutically effective amount of a GHS;
b a therapeutically effective amount of a second drug selected from: a DPP4 antagonist, a thiazolidinedione, a SGLT2 antagonist, metformin and vitamin E; and,
c a pharmaceutically acceptable carrier;
for use in treating NAFLD and/or NASH.

13. A drug composition, comprising:
a a therapeutically effective amount of a GHS;
b a therapeutically effective amount of a second drug selected from: a DPP4 antagonist, a thiazolidinedione, and a SGLT2 antagonist;
c a therapeutically effective amount of a third drug, which is metformin; and,
d a pharmaceutically acceptable carrier;
for use in treating NAFLD and/or NASH.

14. The drug composition of Claim 12 or 13, wherein the GHS is ibutamoren.

15. The drug composition of Claim 14, wherein the therapeutically effective amount of ibutamoren is 25-50 mg/day.
